# EUROPÄISCHE PATENTANMELDUNG

(11) **EP 1 867 712 A1**
(43) Veröffentlichungstag der Anmeldung: **19.12.2007**
(21) Anmeldenummer: 07109166.4
(22) Anmeldetag: 30.05.2007
(51) Int. Cl.: C12M 1/107

(54) **Biogasreaktor**

(30) Priorität: 14.06.2006 DE 102006027929
(71) Anmelder: AGROTEL GmbH, 94152 Neuhaus am Inn (DE)
(72) Erfinder: Laner, Cyriak, 94152, Neuhaus am Inn (DE)
(74) Vertreter: Benninger, Johannes

(57) **Zusammenfassung**

Die vorliegende Erfindung betrifft ein Verfahren / eine Vorrichtung zur gasdichten Abdichtung von Biogasreaktoren mit zusätzlicher Bereitstellung einer vergrößerten Besiedlungsoberfläche für die Ansiedlung von Mikroorganismen,

Eine besondere Schutzschicht, die zur gasdichten Abdichtung des Innenbereiches von Biogasanlagen dient, weist auf der dem Beton zugewandten Seite eine strukturierte Oberfläche auf. Diese Schutzschicht wird im Gasraum des Reaktors auf die glatte Seite des Beton- Schutzfolien- Verbundes angebracht, so dass die strukturierte Oberfläche in den Reaktorinnenraum weist. Damit ergibt sich eine vergrößerte Oberfläche im Reaktorinnenraum, die die Ansiedlung von Mikroorganismen, insbesondere von entschwefelnden Mikroorganismen erleichtert.

## Beschreibung

Die vorliegende Erfindung betrifft ein Verfahren / eine Vorrichtung zur gasdichten Abdichtung von Biogasreaktoren mit zusätzlicher Bereitstellung einer vergrößerten Besiedlungsoberfläche für die Ansiedlung von Mikroorganismen mit den Merkmalen des Oberbegriffs des Anspruchs 1.

### Stand der Technik

Im Inneren eines Biogasreaktors entsteht als Bestandteil des Biogases unter anderem hochgiftiger Schwefelwasserstoff (H₂S), der außerdem sehr korrosiv ist. Aus diesem Grund muss das entstehende Biogas entschwefelt werden. Die Entschwefelung dient somit der Entfernung eines hochgiftigen Gases und der Vermeidung der Korrosion von Bestandteilen der Anlage.

Zur Verminderung des Schwefelwasserstoffgehalts im Biogas gibt es verschiedene Möglichkeiten. Hierbei hat sich vor allem ein biotechnologisches Verfahren gegenüber bestehenden chemischen Verfahren durchgesetzt, bei dem von einer besonderen Fähigkeit des Bakteriums *Sulfobacter oxydans* und / oder anderer Schwefelbakterien Gebrauch gemacht wird. Das Bakterium *Sulfobacter oxydans* lebt von Schwefelwasserstoff und wandelt diesen in Gegenwart von Sauerstoff in elementaren Schwefel um. Da diese Bakterien omnipräsent sind, müssen sie dem System im Allgemeinen nicht künstlich zugeführt werden. Der Schwefel fällt als gelblicher Belag aus und wird bei der Ausbringung als Pffanzennährstoff genutzt.

Neben H₂S benötigen die eben genannten Bakterien Kohlenstoff, anorganische Salze sowie Spurenelemente. Diese Substanzen liegen im Fermenter in ausreichendem Umfang vor. Weiterhin benötigen diese Mikroorganismen Sauerstoff und eine ausreichend feuchte Oberfläche, da sie nicht im Biogas selbst arbeiten. Diese Voraussetzungen müssen künstlich geschaffen werden. Erweisen sich die Flächen im Gasraum des Fermenters oder im Nachgärbehälter als ungeeignet (zu kleine Fläche bzw. unzureichende Nährstoffversorgung), muss die bereitstehende Oberfläche künstlich vergrößert werden.

Die Gebrauchsmusterschrift DE 202 02 722 U1 beschreibt eine Vorrichtung zum Entschwefeln von Biogas mithilfe von Mikroorganismen, bei der eine Ansiedelfläche für die Mikroorganismen zwischen einem Gas- Einlass und einem Gas- Auslass angeordnet ist und von dem zu entschwefelnden Biogas überströmt wird, wobei der im Biogas enthaltene Schwefelwasserstoff durch die Mikroorganismen abgebaut wird, und bei dem in vorgebbaren Zeitabständen die Ansiedelfläche mit einer Mikroorganismen - enthaltenden Flüssigkeit benetzt wird.

Der Nachteil des Systems, welches in DE 202 02 722 U1 beschrieben wird, besteht darin, dass die Ansiedelfläche häufig, beispielsweise mehrmals täglich mit der Mikroorganismen - enthaltenden Flüssigkeit benetzt werden muss und außerdem in regelmäßigen Abständen, beispielsweise einmal wöchentlich, eine spezielle Nährlösung zugeführt werden muss, da die Entschwefelung in einem Extra - Entschwefelungsreaktor stattfindet.

### Beschreibung

Das Ziel der Erfindung besteht darin, ein Verfahren / eine Vorrichtung zur gasdichten Abdichtung von Biogasreaktoren herzustellen, bei der die Entschwefelung des Biogases innerhalb des Reaktors erfolgt, da eine verbesserte Oberfläche zur Ansiedlung von Mikroorganismen zur Verfügung gestellt wird.

Dieses Ziel der Erfindung wird mit dem Gegenstand des unabhängigen Anspruchs erreicht. Merkmale vorteilhafter Weiterbildungen der Erfindung ergeben sich aus den abhängigen Ansprüchen.

Bei der Erfindung handelt es sich um eine besondere Schutzfolie für den Innenbereich von Biogasanlagen. Die Folie dient zum einen dem Schutz der Flächen innerhalb von Fermentern und Nachgäranlagen, um nachhaltige Schäden an der Baustruktur zu vermeiden. Die Fermenter / Nachgäranlagen bestehen hierbei zumeist aus Beton, es ist aber auch die Verwendung von anderen Baumaterialien denkbar, wobei zusätzlich zwischen diese Baumaterialien und der Schutzfolie eine dünne Betonschicht eingegossen wird.

Die erfindungsgemäße Schutzfolie besteht aus einer Kunststoffschicht, beispielsweise einer 1,5mm dicken PP Folie, auf die ein spezielles Gewebe aufvulkanisiert wird. Dieses Gewebe besteht beispielsweise aus Kunststoff, insbesondere PP, und weist auf einer Seite eine strukturierte Oberfläche auf, die für die Betonhaftung erforderlich ist. Bei der strukturierten Oberfläche handelt es sich z. B. um ein 10 mm tiefes Schlingengewebe, es sind aber auch kleinere oder größere Schlingen, noppenartige Strukturen oder anderweitige Strukturierungen denkbar, bei denen die Oberfläche vergrößert ist, so dass ein fester Verbund zwischen der strukturierten Schutzfolienoberfläche und dem Beton entsteht.

Die Verwendung der erfindungsgemäßen Schutzfolie als Verbundmatte in einer Schalung für Betonwände wird in der Anmeldung DE 10 2004 015 694 A1 offengelegt. Der Offenbahrungsgehalt der Anmeldung DE 10 2004 015 694 A1 wird in diese Anmeldung mit aufgenommen.

Die erfindungsgemäße Schutzfolie weist eine sehr geringe Methan- und Sauerstoffdurchlässigkeit auf, weiterhin zeichnet sie sich durch eine gute Temperaturbeständigkeit (keine Änderung der Struktur bei 60°C und 75% Luftfeuchtigkeit) sowie eine gute chemische Beständigkeit gemäß ISO 175 und eine gute UV - Beständigkeit gemäß DIN 53387 aus. Die Schutzfolie weist eine gute Beständigkeit in Anwesenheit von Schwefelsäure pH 3 und pH1 auf, weiterhin ist sie Gülle und Gasbeständig.

Aufgrund der oben beschriebenen Eigenschaften eignet sich die Schutzfolie hervorragend zum Schutz der Bausubstanz. Der Biogasbehälter wird innenwandig, d.h. im Bereich der Innenwände, der Decken und eventuell auch im Bereich des Bodens, mit der Schutzfolie verkleidet. Dabei ist die strukturierte Oberfläche nach außen gerichtet. Die Folien werden miteinander gas- und geruchsdicht durch eine so genannte Hohlkehle verschweißt. Durch Eingießen von Beton entsteht aufgrund der strukturierten, beispielsweise mit Schlaufen versehenen Oberfläche, ein fester Verbund und es bildet sich ein gas- und geruchsdichter Raum.

Die Festigkeit des oben beschriebenen Verbundes wurde auf Haftzugfestigkeit getestet und hält problemlos einem Haftzug von bis zu 0,95 N/mm² stand, ohne dass sich die Folie vom Beton löst. Dies gilt auch bei einer Umgebungstemperatur von bis zu 60°C.

Die Schutzfolie wird weiterhin verwendet, um größere Besiedlungsoberflächen für die Ansiedlung von Schwefelwasserstoff abbauenden Mikroorganismen, insbesondere Schwefelbakterien, bereitzustellen. Die Schutzfolie wird dabei zusätzlich im Innenraum von Biogasanlagen eingesetzt. In diesen Biogasreaktoren werden organische Stoffe unter Mitwirkung von Mikroorganismen umgesetzt, wobei Biogas erzeugt wird. Eine solche Anlage umfasst einen gasdicht verschließbaren Reaktor.

Im Inneren des Fermenters muss das entstehende Biogas entschwefelt werden, da es zum einen Schwefelwasserstoff (H₂S) enthält und somit hochgiftig ist und zum anderen sehr korrosiv ist. Die Entschwefelung dient also auch dazu, die Korrosion von Bestandteilen der Anlage zu vermeiden.

Der erste Schutzmechanismus für die Baustruktur ist das Verkleiden des Innenraums mit der oben beschriebenen Schutzfolie. Ein zweiter Schutzmechanismus besteht in der biotechnologischen Entschwefelung des Biogases. Dabei wird mithilfe von Bakterien unter gezieltem Einblasen geringer Luftmengen in den Gasraum das Ausfällen von elementarem Schwefel erreicht.

Damit die Entschwefelung bereits im Biogasreaktor stattfindet, benötigen die Mikroorganismen optimale Wachstumsbedingungen. Dazu zählt zum einen das Vorhandensein der nötigen Nährstoffe wie z.B. Kohlenstoffverbindungen, anorganische Salze und Spurenelemente, sowie der benötigte Sauerstoff und eine feuchte Oberfläche, auf der sich die Bakterien ansiedeln können.

Um die Besiedlung der Wandflächen durch Mikroorganismen im oberen Reaktorinnenraum oberhalb des Flüssigkeitsraums zu erleichtern, ist es günstig, die vorhandene Oberfläche zu verbessern, insbesondere die Oberfläche zu vergrößern. Die Vergrößerung der Oberfläche im Biogasreaktorinnenraum wird gemäß vorliegender Erfindung mithilfe einer zweiten Schicht der oben beschriebenen Schutzfolie erreicht. Diese zweite Schicht dient als so genannte Besiedlungsmatte für die zur Entschwefelung wichtigen Bakterien.

Und zwar wird im Innenraum eines Biogasreaktor zumindest teilweise die Besiedlungsfläche für die Mikroorganismen vergrößert.

Zur Verwendung der oben beschriebenen Schutzfolie als Besiedlungsmatte wird eine zweite Folienschicht auf die bereits vorhandene, mit dem Beton verbundene Schutzfolie aufgebracht. Die zweite Schicht kann die Wand- und Deckenreaktorflächen im oberen Gasraum, d.h. oberhalb des Flüssigkeitsraumes, dabei ganz oder auch nur teilweise bedecken.

Die zweite Schicht wird so angebracht, dass die strukturierte Oberfläche dem Innenraum des Biogasreaktors bzw. Fermenters zugewandt ist. Die einander zugewandten glatten Oberflächen der beiden Schutzfolien werden beispielsweise durch Verschweißen oder aufvulkanisieren fest und gasdicht miteinander verbunden. Es ist allerdings auch denkbar, die beiden Folien gasdicht miteinander zu verkleben oder zu vernähen.

Somit wird im Innenraum des Fermenters die Oberfläche, die von den Bakterien besiedelt werden kann, aufgrund der strukturierten Oberfläche erhöht und die Ansiedlung der Bakterien erleichtert. Bei den sich ansiedelnden Mikroorganismen handelt es sich insbesondere um Schwefelbakterien, die die Entschwefelung des Biogases durchführen, indem sie den im Biogas enthaltenen Schwefelwasserstoff (H₂S) in elementaren Schwefel umwandeln.

Die innere Folienschicht oder auch Besiedlungsschicht wird hierbei im Allgemeinen nur im oberen Bereich der Anlage oberhalb des Flüssigkeitsraums angebracht.

Gemäß einer weiteren Ausführungsvariante ist aber auch eine vollständige innenwandige Verkleidung mit der zweiten Folienschicht denkbar. Ebenso können aus Gründen der Kostenersparnis auch nur Teilbereiche der Beton - Schutzfolienverbundschicht im oberen Bereich der Wand und im Deckenbereich mit einer zweiten Folienschicht verbunden werden, um vermehrte Besiedlungsflächen zu schaffen.

Gemäß einer weiteren Ausführungsvariante wird innenwandig nicht eine vollständige zweite Schutzfolienschicht aufgebracht, sondern nur eine weitere Gewebeschicht mit strukturierter Oberfläche auf die Schutzfolie aufvulkanisiert, wobei die strukturierte Oberfläche in den Innenraum des Biogasreaktors zeigt. Diese zweite Ausführungsvariante kann wie oben beschrieben vollständig oder teilweise auf die dem Reaktorinnenraum zugewandte glatte Seite der ersten Schutzfolie aufgebracht sein.

Das Verbinden der ersten Schutzschicht mit der zweiten Schutzschicht oder mit der zusätzlichen Gewebeschicht kann erfolgen, nachdem die erste Schutzschicht mit Beton eingegossen und verbunden wurde.

Gemäß einer weiteren Ausführungsvariante wird die Besiedlungsfläche erst mit der ersten Schutzfolie verbunden, anschließend werden die konfektionierten Bahnen der Schutzfolie mit zusätzlichen Besiedlungsoberflächen entsprechend aufgestellt und mit Beton eingegossen, so dass der gasdichte Reaktorinnenraum gebildet wird.

### Figurenbeschreibung

Weitere Merkmale, Ziele und Vorteile der vorliegenden Erfindung gehen aus der nun folgenden detaillierten Beschreibung einer bevorzugten Ausführungsform der Erfindung hervor, die als nicht einschränkendes Beispiel dient und auf die beigefügten Zeichnungen Bezug nimmt. Gleiche Bauteile weisen dabei grundsätzlich gleiche Bezugszeichen auf und werden teilweise nicht mehrfach erläutert.
Fig. 1 zeigt die Verwendung der Betonschutzfolie in einem Biogasreaktor, wobei zumindest teilweise eine zweite Schicht Schutzfolie oder strukturiertes Gewebe zur Vergrößerung der Besiedlungsfläche aufgebracht ist;
Fig. 2 zeigt den Aufbau der Schutzfolie,
Fig. 3 zeigt eine mögliche Ausführungsform, bei der zwei Schutzfolien miteinander verbunden wurden, so dass die strukturierten Oberflächen jeweils nach außen zeigen,
Fig. 4 zeigt eine Ausführungsform, bei der eine weitere strukturierte Gewebeschicht auf die glatte Oberfläche einer Schutzfolie gemäß Figur 2 aufgebracht wurde,
Fig. 5 zeigt die gasdichte Verschweißung zwischen Wand- und Deckenschutzfolie im Bauzustand,
Fig. 6 zeigt die gasdichte Verschweißung von Wand-, Stützenpilz- und Aussparungsöffnung,
Fig. 7 zeigt ein weiteres Ausführungsbeispiel eines Biogasreaktors mit einer Folien - Dachkonstruktion und
Fig. 8 zeigt den Wandanschluss ein einem erfindungsgemäßen Betonbehälter.

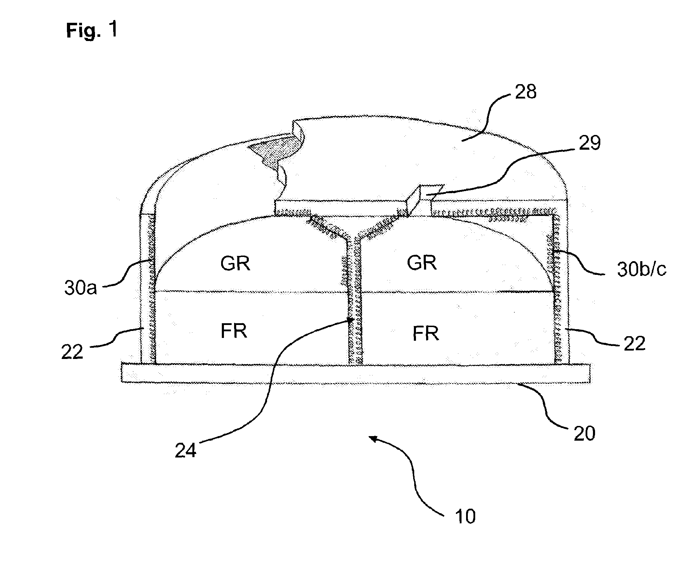

Eine mögliche Ausgestaltung eines Biogasreaktors 10 mit erfindungsgemäßer abdichtender Schutzfolie 30, die zusätzlich noch eine erhöhte Besiedlungsoberfläche für die Ansiedlung von Schwefelbakterien aufweist, wird anhand von Figur 1 illustriert.

Der Fermenter bzw. Biogasreaktor 10 besteht aus einer flüssigkeitsdichten Betonfußplatte 20, welche beispielsweise kreisförmig gestaltet sein kann, wenn es sich bei dem Reaktor um eine Konstruktion mit kreisförmigem Grundriss handelt. Ebenso sind rechteckige Ausführungsformen denkbar, wobei in dem Fall vielfache Ecken entstehen, die jeweils gasdicht, beispielsweise durch Verschweißen, Verkleben etc. verschlossen werden müssen.

Die Wände 22 des Fermenters 10 bestehen aus einer Beton - Schutzfolie - Verbundschicht 30a, ebenso der mittlere Stützpfeiler 24 in Pilzausführung und die nach oben abschließende Decke 28. Bei dem in Figur 1 dargestellten Biogasreaktor 10 besteht die nach oben abschließende Decke 28 aus Beton (B).

Zur Erleichterung der Wartung der Anlage 10 gibt es mindestens eine Aussparungs- oder Serviceöffnung 29, die mit einer Klappe oder ähnlichem luftdicht verschlossen werden kann.

Bei der Konstruktion der Anlage wird im Innenbereich eine Schutzfolie 30a in vorbereiteter Größe aufgestellt und anschließend die äußere Betonwand 22 mit der Schutzfolie 30a durch Eingießen von Beton miteinander verbunden. Die strukturierte Oberfläche der Schutzfolie 30a erlaubt eine enorm starke Verbindung zwischen den unterschiedlichen Materialien Beton und Kunststoff mit einer hohen Haftzugfestigkeit. Bei dem Material, aus dem die Schutzfolie 30a besteht, handelt es sich um ein Gasdichtes, Säure- und Chemikalien sowie Temperaturbeständiges Material. Dafür eignet sich beispielsweise Kunststoff, wie z.B. Polypropylen.

Der untere Bereich des Reaktors 10 ist im Allgemeinen mit Flüssigkeit befüllt. Diese stellt den Flüssigkeitsraum FR dar. Im oberen Bereich sammeln sich die Biogase im so genannten Gasraum GR.

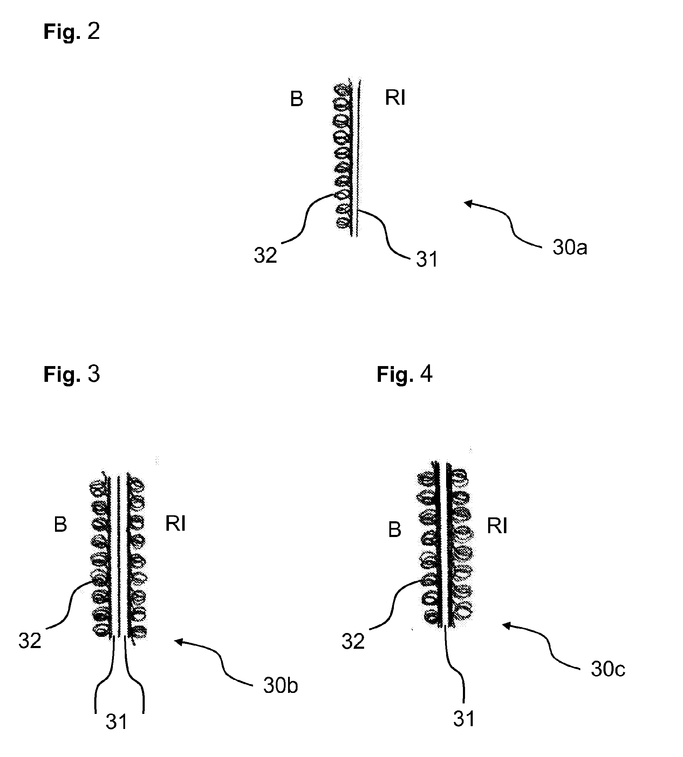

Wie in Figur 2 abgebildet, ist die Schutzfolie 30a zweischichtig aufgebaut. Die Schicht 31 besteht im Ausführungsbeispiel aus einer Polypropylenfolie einer Stärke von ca. 1,5 bis 2 mm. Auf diese Kunststoffschicht ist eine strukturierte Gewebeschicht 32 aufgebracht.

Bei den Strukturen handelt es sich im Ausführungsbeispiel um Schlingen mit einem Durchmesser von ungefähr 10 mm. Gemäß einer weiteren Ausführung der Erfindung wird die Oberfläche durch das Vorhandensein von Noppen gekennzeichnet. Die strukturierte Gewebeschicht 32 besteht im Ausführungsbeispiel ebenfalls aus Propylen, es sind aber auch andere umweltbeständige Materialien denkbar.

Die strukturierte Oberfläche 32 ist im Falle des Verbundes zwischen Schutzfolie 30a und Beton B zum Beton B hin gerichtet. Die glatte Oberfläche 31 weist in Richtung Reaktorinnenraum RI.

Zur verbesserten Ansiedlung von Schwefelbakterien im Gasraum des Bioreaktors, wird dieselbe Schutzschicht 30a im Innenraum des Reaktors RI auf die mit dem Beton verbundene Schutzschicht 30a aufvulkanisiert, dadurch entsteht die so genannte Schutzschicht mit zusätzlicher Besiedlungsfläche 30b.

Figur 3 zeigt eine Variante der Schutzschicht mit zusätzlicher Besiedlungsfläche 30b, bei der eine zweite Schutzfolie 30a, bestehend aus der tragenden Schicht 31 und der Gewebeschicht 32, so mit der erste Schutzfolie 30a verbunden wurde, so dass die strukturierten Oberflächen nach außen weisen. Die Befestigung der beiden Folien miteinander erfolgt durch Verschweißen, verkleben, vulkanisieren etc.

Figur 4 zeigt eine weitere erfindungsgemäße Ausführungsform 30c, bei der eine zweite Gewebeschicht 32 auf die tragende Schicht 31 der ersten Schutzschicht 30a aufgebracht wurde, so dass die strukturierten Oberflächen wiederum nach außen weisen.

Gemäß den in den Figuren 3 und 4 beschriebenen Ausführungsformen erhält man im Innenraum des Biogasreaktors 10 eine erhöhte Oberfläche, auf der die Ansiedlung Schwefelwasserstoff abbauender Bakterien erleichtert ist.

Die Schutzschicht mit zusätzlicher Besiedlungsfläche 30b oder 30c wird vor allem im Bereich des Gasraums des Bioreaktors verwendet. Hierbei ist sowohl eine vollständige als auch eine teilweise Abdeckung der Wand- bzw. Deckenfläche mit zusätzlicher Besiedlungsfläche 30b/c denkbar.

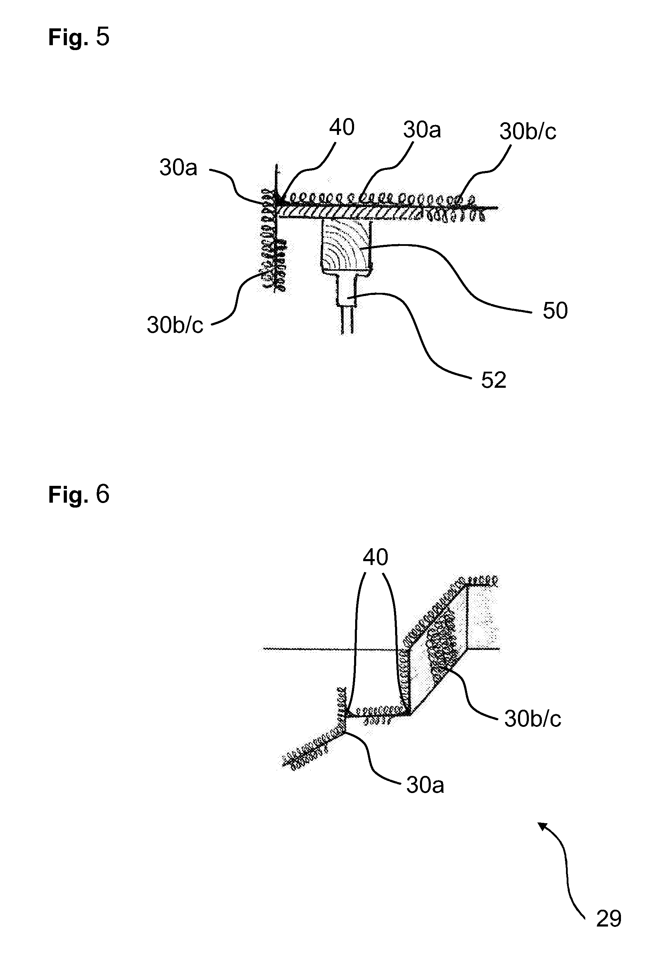

Figur 5 zeigt eine gasdichte Verschweißung von Wand- und Deckenfolie 30a sowie teilweise 30b oder 30c im Bauzustand. Das Verbinden der Schutzschichten untereinander an der glatten Schicht 31 erfolgt mittels einer Hohlkehlnaht 40 durch Verschweißen. Der Einbau der Betonschutzfolie in die Schalung erfolgt hierbei vor der Betonierung. Solange der Beton noch nicht getrocknet und ein starrer Verbund hergestellt ist, muss der Innenraum des Reaktor mit einer Holzschalung 50 und entsprechender Unterkonstruktion 52 abgestützt werden.

Figur 6 zeigt die gasdichte Verschweißung im Bereich von Wand 22 bzw. Dach 28, Stützenpilz 24 und Aussparungs- bzw. Serviceöffnung 29. Hierbei finden wiederum Hohlkehlnähte 40 Verwendung.

Gemäß einer bevorzugten Ausführungsform wird die zweite Schutzschicht 30a oder die zweite Gewebeschicht 32 bereits vor dem Eingießen des Betons mit der ersten Schutzschicht verbunden.

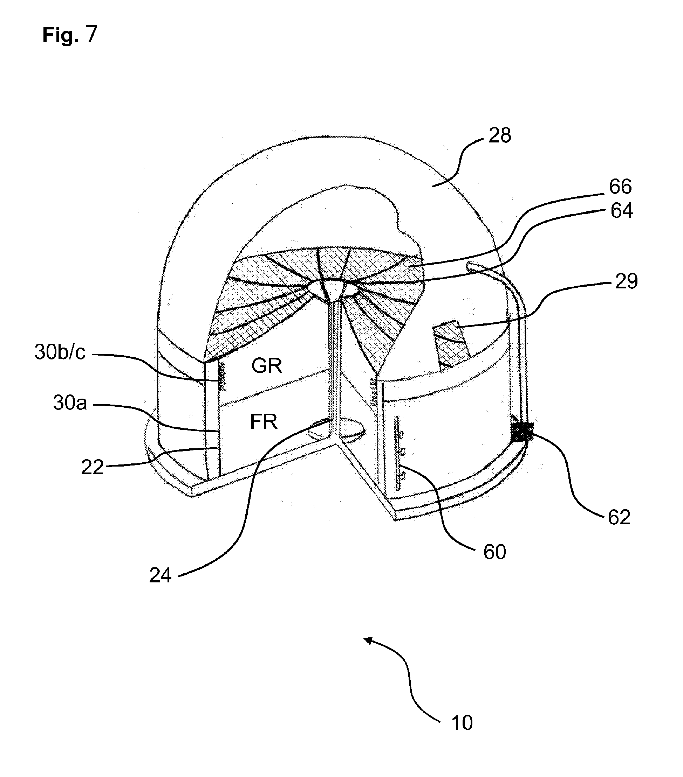

Figur 7 zeigt eine weitere mögliche Ausgestaltung eines Biogasreaktors 10, bei welchem als nach oben abschließende Decke 28 anstelle eines Betondachs eine Doppelmembran Verwendung findet. In das Doppelmembran - Dach kann ebenfalls ein luftdicht verschließbarer Serviceschacht 29 eingearbeitet werden, um bei später eventuell nötigen Servicearbeiten einen verbesserten Zugang zum Innenbereich des Reaktors zu haben.

Der in Figur 7 dargestellte erfindungsgemäße Doppelmembran - Gasspeicher enthält mittig einen mittleren Stützpfeiler 24, der beispielsweise aus Edelstahl V2A, V4A oder einem anderen beständigen und stabilen Material besteht.

Vom Stützpfeiler 24 ausgehend ziehen sich Gurte 64 zu den Wänden 22 des Biogasreaktors 10, die ein vollflächig verlegtes Netz 66 stabilisieren. Über diesem Netz 66wölbt sich eine kuppelförmige Dachkonstruktion, die im erfindungsgemäßen Ausführungsbeispiel aus einer Doppelmembran besteht.

Um die Menge des Gases im Gasspeicher 10 überprüfen zu können, befindet sich im Außenwandbereich eine Füllstandsanzeige mit einem Magnetschalter 60.

Weiterhin weist der in Figur 7 dargestellte Reaktor ein externes Stützgebläse mit Druckwächter 62 auf, um die Außenmembran in einer witterungsunabhängigen, stabilen Form zu halten.

Die Betonwände im Inneren des Speichers werden wiederum durch Verwendung der Betonschutzfolie 30a geschützt. Die Schutzfolie bildet über ihre Seite mit der strukturierten Oberfläche zusammen mit dem eingegossenen Beton einen festen Verbund. Auf der Wandinnenfläche wird im Gasraum des Speichers eine zweite Schicht der Betonschutzfolie 30a bzw. der Gewebeschicht mit der strukturierten Oberfläche 32 aufgeschweißt, so dass die strukturierte Oberfläche in den Innenraum weist und somit eine erhöhte Besiedlungsfläche 30b/c für Mikroorganismen bildet.

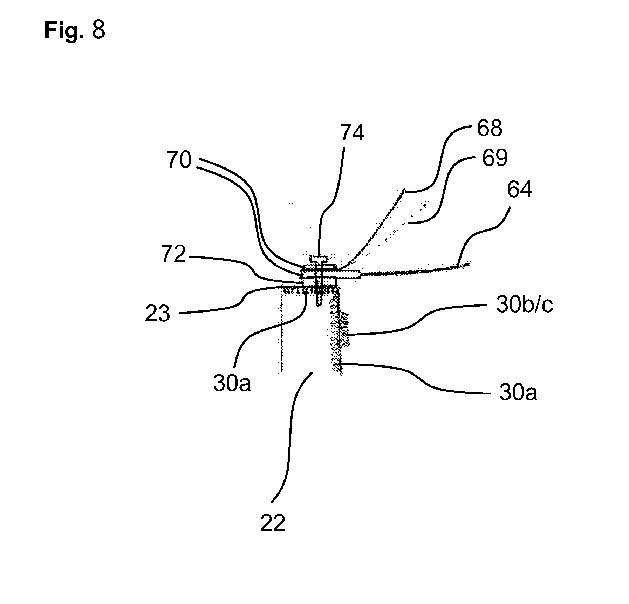

Figur 8 zeigt den Wandanschluss in einem erfindungsgemäßen Betongasspeicher 10, Die Wände 22 und die Wandkrone 23 des Speichers werden durch einen starren Verbund aus Betonschutzfolie 30a und Beton gebildet. Darauf befindet sich zumindest teilweise eine zweite Schicht der Betonschutzfolie 30a bzw. der Gewebeschicht mit der strukturierten Oberfläche 32.

Auf die Wände 22 wird zum einen das vollflächig verlegte Netz sowie die gasdicht abschließende Doppelmembran aufmontiert. Die Montage erfolgt im Ausführungsbeispiel im Bereich der Wandkrone 23, die ebenfalls aufgrund des stabilen Verbundes mit Betonschutzfolie 30a vor Korrosion etc. geschützt ist. Über einer dicht abschließenden Zellkautschukdichtung 72 befinden sich Stahlprofile 70, die sowohl der Befestigung der das Netz 66 haltenden Gurte 64 als auch der Befestigung der den Speicher oben abschließenden Membranen dient. Es werden sowohl die innere Membran 69 als auch die äußere Membran 68 auf diese Weise befestigt. Diese Elemente werden zusammen mit einer Schraube 74 oder einem anderen Befestigungselement am Schutzfolien 30a - Beton - Verbund fixiert.

Die Erfindung ist nicht auf die vorstehenden Ausführungsbeispiele beschränkt. Vielmehr ist eine Vielzahl von Varianten und Abwandlungen denkbar, die von dem erfindungsgemäßen Gedanken Gebrauch machen und deshalb ebenfalls in den Schutzbereich fallen.

### Bezugszeichenliste

- B: Beton
- FR: Flüssigkeitsraum
- GR: Gasraum
- RI: Reaktorinnenraum

- 10: Biogasreaktor / Fermenter
- 20: Betonfußplatte
- 22: Wände des Biogasreaktors
- 23: Wandkrone
- 24: Stützpfeiler
- 28: Decke
- 29: Serviceöffnung
- 30a: Schutzfolie / Schutzschicht
- 30b: Schutzfolie mit zusätzlicher Besiedlungsschicht
- 30c: Schutzfolie mit zusätzlicher Besiedlungsschicht
- 31: Folienschicht
- 32: strukturierte Oberfläche
- 40: Hohlkehlnaht
- 50: Holzschalung
- 52: Unterkonstruktion
- 60: Magnetschalter
- 62: Druckwächter
- 64: Gurte
- 66: Netz
- 68: äußere Membran
- 69: innere Membran
- 70: Stahlprofil
- 72: Zellkautschukdichtung
- 74: Schraube / Befestigungselement

## Patentansprüche

1. Biogasanlage zur Behandlung organischer Stoffe unter Mitwirkung von Mikroorganismen die insbesondere die Erzeugung von Biogas bewirken, wobei die Anlage einen gasdicht verschließbaren Reaktor umfasst, **dadurch gekennzeichnet, dass** der Innenraum des Reaktors (RI) zumindest teilweise eine vergrößerte Besiedlungsfläche für Mikroorganismen aufweist.

2. Biogasanlage gemäß Anspruch 1, **dadurch gekennzeichnet, dass** die sich ansiedelnden Mikroorganismen Schwefelbakterien sind, die die Entschwefelung des Biogases durchführen, indem sie den im Biogas enthaltenen Schwefelwasserstoff (H₂S) in elementaren Schwefel umwandeln.

3. Biogasanlage gemäß Anspruch 1 und 2, **dadurch gekennzeichnet, dass** die Besiedlungsfläche eine strukturierte Oberfläche, insbesondere ein Schlaufengewebe (32) ist.

4. Biogasanlage gemäß einem der vorangehenden Ansprüche, **dadurch gekennzeichnet, dass** die strukturierte Oberfläche an der Reaktorinnenseite oberhalb des Flüssigkeitsraums (FR) angebracht ist und diesen Teil der Reaktorwandfläche ganz oder teilweise bedeckt.

5. Verfahren zur Herstellung einer vergrößerten Besiedlungsfläche zur Verwendung in einer Biogasanlage gemäß einem der Ansprüche 1 bis 4, **dadurch gekennzeichnet, dass** eine strukturierte Oberfläche mit einer Schutzschicht (30a), die zusammen mit Beton eine gasdichte Wand des Biogasreaktors bildet, im Reaktorinnenraum (Rl) verbunden wird.

6. Verfahren gemäß Anspruch 5, **dadurch gekennzeichnet, dass** dieselbe Schutzschicht (30a), die zum Schutz der Reaktorinnenwände verwendet wird, zumindest teilweise auf die Wand- und / oder Deckeninnenseite im Inneren des Bioreaktors aufgebracht wird, so dass eine doppelte Schutzschicht (30b) entsteht, wobei die strukturierte Oberfläche der zweiten, inneren Schutzschicht (30a) in den Reaktorinnenraum (Rl) weist.

7. Verfahren gemäß Anspruch 5, **dadurch gekennzeichnet, dass** eine strukturierte Gewebeschicht (32) zumindest teilweise auf die Wand- und / oder Deckeninnenseite im Inneren des Bioreaktors aufgebracht wird, so dass eine Schutzschicht (30c) entsteht, wobei die strukturierte Oberflächenseite der Gewebeschicht (32) in den Reaktorinnenraum (RI) weist.

8. Verfahren gemäß einem der Ansprüche 5 bis 7, **dadurch gekennzeichnet, dass** die Besiedlungsschicht durch Verschweißen, Verkleben, Vulkanisieren oder Vernähen mit dem Beton - Schutzfolien (30a) - Verbund verbunden wird oder dass die Besiedlungsschicht durch Verschweißen, Verkleben, Vulkanisieren oder Vernähen mit der Schutzfolie (30a) verbunden wird, bevor diese durch Eingießen von Beton in die Reaktorwand verbaut wird.

9. Besiedlungsmatte zur verbesserten Ansiedlung von Mikroorganismen insbesondere in einem Biogasreaktor gemäß wenigstens einem der Ansprüche 1 bis **4, dadurch gekennzeichnet, dass** sie eine Besiedlungsfläche in Form einer strukturierten Oberfläche, insbesondere in Form eines Schlaufengewebe (32) aufweist.

10. Besiedlungsmatte gemäß einem der vorangehenden Ansprüche, **dadurch gekennzeichnet, dass** die Besiedlungsfläche an der Reaktorinnenseite eines Fermenters oberhalb des Flüssigkeitsraums (FR) angebracht ist und diesen Teil der Reaktorwandfläche ganz oder teilweise bedeckt.
